# EUROPEAN PATENT APPLICATION

(11) **EP 3 097 842 A1**
(43) Date of publication of application: **30.11.2016**
(21) Application number: 15822281.0
(22) Date of filing: 29.06.2015
(51) Int. Cl.: A61B 1/00, G02B 23/24

(54) **INSERTION DEVICE**

(30) Priority: 17.07.2014 JP 2014147066
(71) Applicant: Olympus Corporation, Tokyo 192-8507 (JP)
(72) Inventor: ONODA, Fumiyuki, Hachioji-shi, Tokyo 192-8507 (JP); YAMASHITA, Takashi, Hachioji-shi, Tokyo 192-8507 (JP); OMOTO, Keijiro, Hachioji-shi, Tokyo 192-8507 (JP); MIYAKE, Kensuke, Hachioji-shi, Tokyo 192-8507 (JP); HATA, Kazuhiro, Hachioji-shi, Tokyo 192-8507 (JP)
(74) Representative: Gunzelmann, Rainer
(86) International application number: PCT/JP2015/068657
(87) International publication number: WO 2016/009819

(57) **Abstract**

An insertion apparatus includes an insertion portion 10, a power spiral tube 17 provided with a spiral fin portion 18 configured to extend in a spiral shape, the power spiral tube 17 being provided on an outer circumferential direction side of the insertion portion 10 so as to be rotatable with respect to the insertion portion 10 around a longitudinal axis, a first motor 23 configured to generate a drive force to rotate the power spiral tube 17, an operation section 11 provided on a proximal end side of the insertion portion 10, a second motor 27 configured to generate a drive force to rotate the insertion portion 10 pivotably held to the operation section 11, a first motor drive circuit 33 configured to calculate a current value to drive the first motor 23, a torque detection circuit 34 configured to calculate torque to be added to the power spiral tube 17 from the calculation result, and a second motor drive circuit 35 configured to perform control so as to supply a current that causes the second motor 27 to rotate in accordance with the calculation result.

## Description

### Technical Field

The present invention relates to an insertion apparatus, and more particularly, to an insertion apparatus whose insertion portion is provided with a power spiral tube.

### Background Art

Conventionally, endoscope systems provided with an endoscope that picks up images of an object inside a subject and a video processor that generates an observed image of the object picked up by the endoscope, or the like have been widely used in a medical field, an industrial field, and the like.

As such an endoscope, Japanese Patent Application Laid-Open Publication No. 2008-272302 discloses a power spiral endoscope provided with an insertion portion to which a rotation unit having a power spiral tube is attached so as to be rotatable around a longitudinal axis.

A conventional power spiral endoscope displays torque received by the power spiral tube while being inserted into the body by the number of LEDs, which are "ON," provided in an external display unit. More specifically, as the torque received by the power spiral tube increases, the number of LEDs which are "ON" is increased to allow the operator to recognize the torque received by the power spiral tube.

However, the operator cannot realize the torque actually received by the power spiral tube only by displaying the torque by the number of LEDs which are "ON" as in the case of the conventional external display unit.

The torque received by the power spiral tube is transmitted to the operator who is grasping an operation section via the insertion portion of the endoscope, but the torque transmitted does not always coincide with the torque actually received by the power spiral tube.

For that reason, the operator cannot intuitively realize the torque received by the power spiral tube, and so the operator needs to carefully operate the power spiral tube, resulting in poor operability.

It is therefore an object of the present invention to provide an insertion apparatus that allows the operator to intuitively realize the torque received by the power spiral tube.

### Disclosure of Invention

### Means for Solving the Problem

An insertion apparatus according to an aspect of the present invention includes an insertion portion configured to extend along a longitudinal axis from a proximal end direction to a distal end direction, a rotation unit provided with a spiral fin portion configured to extend along the longitudinal axis in a spiral shape, the rotation unit being provided on an outer circumferential direction side of the insertion portion so as to be rotatable with respect to the insertion portion around the longitudinal axis, a rotation unit driving member configured, by being driven, to generate a drive force to rotate the rotation unit, an operation section provided on a proximal end side of the insertion portion, an insertion portion driving member configured to generate a drive force to rotate the insertion portion pivotably held to the operation section, a drive current calculation section configured to calculate a current value for driving the rotation unit driving member, a torque calculation section configured to calculate torque to be added to the rotation unit from a calculation result of the drive current calculation section, and a control section configured to perform control so as to supply a current to rotate the insertion portion driving member according to the calculation result of a torque calculation section.

### Brief Description of the Drawings

Fig. 1 is a diagram illustrating an overall configuration of an insertion apparatus according to an embodiment;
Fig. 2 is a diagram for describing a detailed configuration of an endoscope;
Fig. 3 is a cross-sectional view for describing a configuration of portion A shown in Fig. 2;
Fig. 4 is a diagram for describing a circuit configuration of a power spiral controller; and
Fig. 5 is a flowchart for describing operation of the insertion apparatus.

### Best Mode for Carrying Out the Invention

Hereinafter, an embodiment of the present invention will be described with reference to the accompanying drawings.

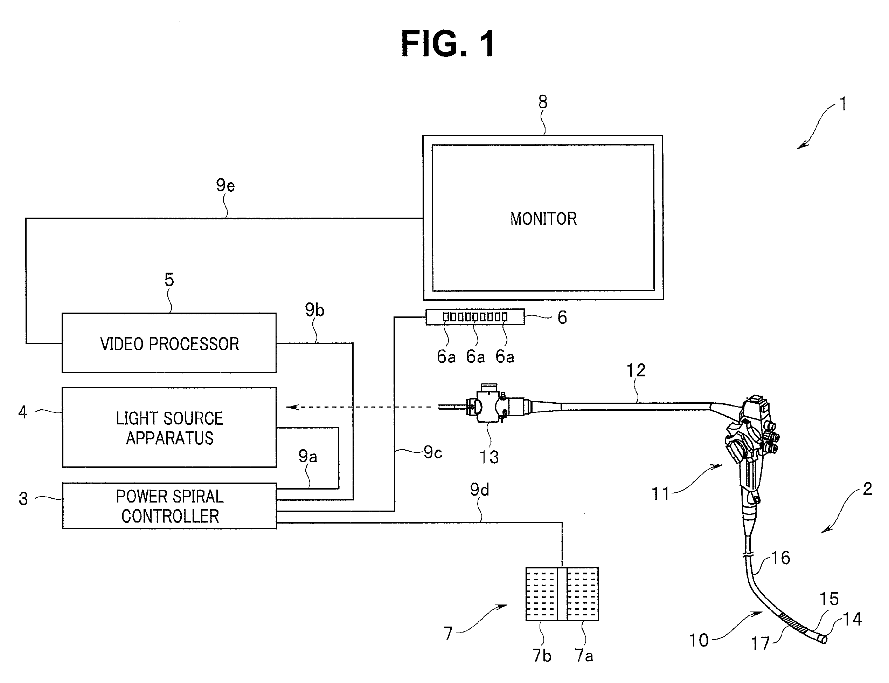

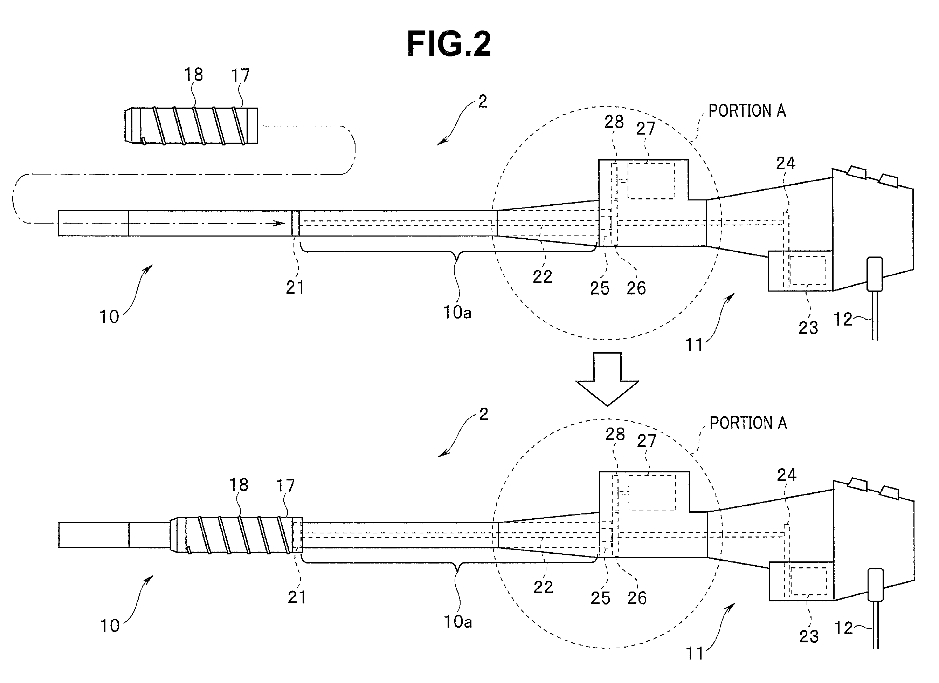

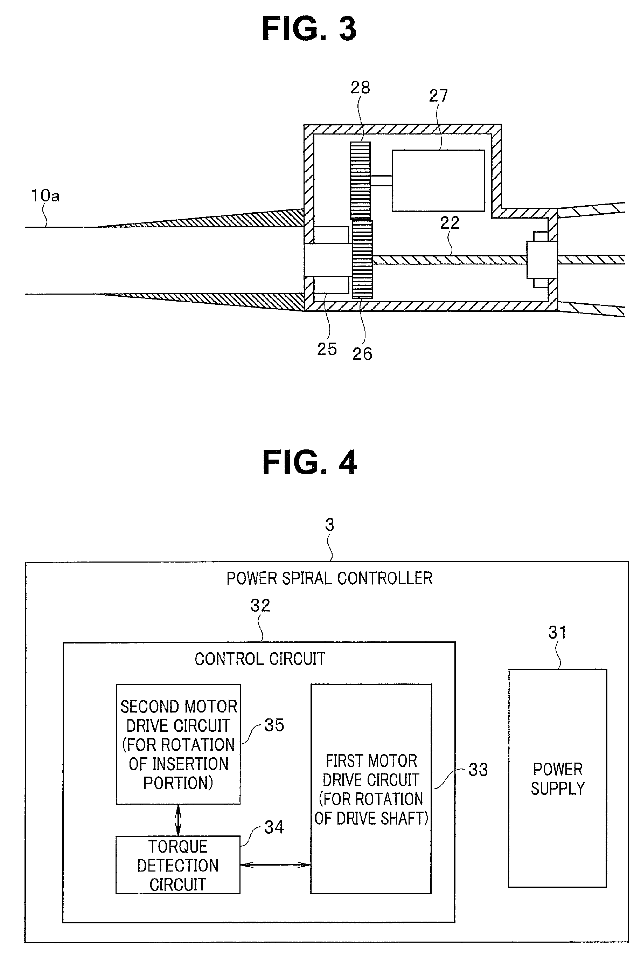

First, a configuration of an insertion apparatus according to an embodiment will be described using Fig. 1 to Fig. 4. Fig. 1 is a diagram illustrating an overall configuration of the insertion apparatus according to the embodiment, Fig. 2 is a diagram for describing a detailed configuration of the endoscope, Fig. 3 is a cross-sectional view for describing a configuration of portion A shown in Fig. 2, and Fig. 4 is a diagram for describing a circuit configuration of a power spiral controller.

As shown in Fig. 1, an endoscope system 1 which is an insertion apparatus is constructed of a power spiral endoscope (hereinafter simply referred to as "endoscope") 2, a power spiral controller 3, a light source apparatus 4, a video processor 5, an external display unit 6, a foot switch 7 and a monitor 8.

The power spiral controller 3 is connected to the light source apparatus 4, the video processor 5, the external display unit 6 and the foot switch 7 via cables 9a, 9b, 9c and 9d respectively. The video processor 5 is connected to the monitor 8 via a cable 9e.

The endoscope 2 is constructed of an insertion portion 10 configured to be inserted into a body cavity, an operation section 11 provided on a proximal end side of the insertion portion 10, a universal cord 12 which is an electric cable extending from one side face of the operation section 11, and a connector portion 13 disposed at an extending end of the universal cord 12. The connector portion 13 is connected to the light source apparatus 4. Illuminating light from the light source apparatus 4 is guided up to a distal end of the insertion portion 10 by an illumination light guide, which is not shown and inserted into the endoscope 2, to illuminate the object.

The insertion portion 10 extends from a proximal end direction to a distal end direction along the longitudinal axis and is constructed of a distal end portion 14, a bending portion 15 and a flexible tube portion 16, connected consecutively in order from the distal end side. An image pickup device, which is not shown and configured to pick up an image of the object is provided inside the distal end portion 14. The image pickup device picks up an image of the object illuminated with illuminating light from the light source apparatus 4 and outputs an image pickup signal. The image pickup signal outputted from the image pickup device is inputted to the video processor 5 via the light source apparatus 4, the cable 9a, the power spiral controller 3 and the cable 9b.

The video processor 5 outputs an image signal obtained by applying predetermined image processing to the inputted image pickup signal to the monitor 8 via the cable 9e. This allows the endoscope image picked up at the endoscope 2 to be displayed on the monitor 8.

The operation section 11 is provided with a bending operation knob configured to bend the bending portion 15 and operation buttons to perform various operations (air feeding, water feeding or the like) of the endoscope 2.

Furthermore, a power spiral tube 17 is configured to be attachable at a predetermined position of the insertion portion 10. More specifically, the power spiral tube 17 is attached to a rotator 21 shown in Fig. 2. The predetermined position where the power spiral tube 17 is attached is, for example, the bending portion 15, but may also be the distal end portion 14 or the flexible tube portion 16.

The power spiral tube 17 is configured so as to be rotatable around the axis in the insertion direction. The power spiral tube 17 is provided with a spiral fin portion 18 on an outer circumferential face as shown in Fig. 2. When the power spiral tube 17 rotates, the spiral fin portion 18 on the outer circumferential face comes into contact with an inner wall of the body cavity of the subject, thereby produces thrust, and the power spiral tube 17 itself is urged to move toward the insertion direction or toward a direction opposite to the insertion direction. Thus, the power spiral tube 17 is provided with the spiral fin portion 18 configured to extend in a spiral shape along the longitudinal axis, thus constituting a rotation unit provided on the outer circumferential direction side of the insertion portion 10 so as to be rotatable with respect to the insertion portion 10 around the longitudinal axis.

The power spiral controller 3 controls driving of the power spiral tube 17 in response to an operation signal from the foot switch 7 that constitutes a rotation operation section. For example, when an F (forward) pedal 7a of the foot switch 7 is pressed down by an operator such as a medical doctor, the power spiral tube 17 rotates in a predetermined direction under the control of the power spiral controller 3 and the power spiral tube 17 itself moves toward the insertion direction.

On the other hand, when a B (backward) pedal 7b of the foot switch 7 is pressed down by the operator, the power spiral tube 17 rotates in a direction opposite to the predetermined direction under the control of the power spiral controller 3 and the power spiral tube 17 itself moves toward a direction opposite to the insertion direction.

Furthermore, the power spiral controller 3 outputs a control signal to the external display unit 6 via the cable 9c so as to display a level of torque on the external display unit 6 in accordance with the operation on the foot switch 7. The external display unit 6 is provided with a plurality of LEDs 6a and configured to display the level of torque in a meter in accordance with a control signal from the power spiral controller 3 by the number of LEDs 6a which are "ON."

More specifically, when the foot switch 7 is not pressed down, the external display unit 6 switches ON only the LED 6a at the center. While the F pedal 7a of the foot switch 7 is being pressed down, the external display unit 6 increases the number of LEDs 6a which are "ON" from the center to the right in accordance with the amount of current flowing through a first motor 23 shown in Fig. 2. On the other hand, while the B pedal 7b of the foot switch 7 is being pressed down, the external display unit 6 increases the number of LEDs 6a which are "ON" from the center to the left in accordance with the amount of current flowing through the first motor 23 shown in Fig. 2.

As shown in Fig. 2, the rotator 21 configured to produce thrust on the power spiral tube 17 is provided at a predetermined position of the insertion portion 10. The power spiral tube 17 engages with this rotator 21.

A drive shaft 22 is inserted through the insertion portion 10 and the operation section 11 of the endoscope 2 and the rotator 21 engages with a distal end portion (insertion portion 10 side) of the drive shaft 22.

The first motor 23 for rotating the drive shaft 22 and a gear 24 of the first motor 23 are disposed in the operation section 11. The gear 24 of the first motor 23 engages with a proximal end portion (operation section 11 side) of the drive shaft 22 and a rotation drive force of the first motor 23 is transmitted to the drive shaft 22. This rotation drive force is transmitted to the power spiral tube 17 via the rotator 21, whereby the power spiral tube 17 is driven to rotate.

In the present embodiment, the rotation corresponding to the torque generated when the power spiral tube 17 is driven to rotate is transmitted to the insertion portion 10, whereby the insertion portion 10 is rotated. More specifically, the insertion portion 10 from the rotator 21 to the operation section 11 (reference numeral 10a in Fig. 2) is configured to rotate. When expressing the insertion portion 10 from the rotator 21 to the operation section 11, the insertion portion 10 is expressed as an insertion portion 10a in the following description.

As shown in Fig. 3, a bearing 25, a gear 26, a second motor 27 and a gear 28 are disposed in the operation section 11. The insertion portion 10a is pivotably held to the operation section 11 via the bearing 25, which is a bearing section provided in the operation section 11. The gear 26 which is a gear member is disposed on an end face of the bearing 25. The gear 26 transmits a drive force generated in the second motor 27 to the bearing 25. The gear 28, which is a gear member for driving the insertion portion of the second motor 27 for rotating the insertion portion 10a engages with the gear 26.

As shown in Fig. 4, the power spiral controller 3 is provided with a power supply 31 configured to supply power to each circuit and a control circuit 32. The control circuit 32 is provided with a first motor drive circuit 33, a torque detection circuit 34 and a second motor drive circuit 35.

An operation signal from the foot switch 7 is inputted to the first motor drive circuit 33. The first motor drive circuit 33 that constitutes a drive current calculation section calculates a current value (drive signal) for driving the first motor 23 in accordance with this operation signal. This current value is supplied to the first motor 23 provided in the operation section 11 of the endoscope 2 via the cable 9a, the light source apparatus 4, the connector portion 13 and the universal cord 12.

The first motor 23 transmits a rotation drive force corresponding to this current value to the drive shaft 22 via the gear 24. The rotation drive force transmitted to the drive shaft 22 is transmitted to the power spiral tube 17 via the rotator 21. The power spiral tube 17 is driven to rotate in this way. Thus, the first motor 23 constitutes a rotation unit driving member configured to be driven by a drive signal and generate a drive force for rotating the power spiral tube 17.

When the power spiral tube 17 is driven to rotate and torque is transmitted to the power spiral tube 17, a load on the first motor 23 increases via the rotator 21 and the drive shaft 22. As a result, a current value to be added to the first motor 23 increases in the first motor drive circuit 33 to maintain the rotation.

The torque detection circuit 34 that constitutes a torque calculation section is connected to the first motor drive circuit 33 and calculates the torque added to the power spiral tube 17 based on the current value supplied to the first motor 23. The torque detection circuit 34 outputs the calculated torque to the second motor drive circuit 25. Furthermore, the torque detection circuit 34 outputs a control signal to the external display unit 6 based on the detected current value to control the number of LEDs 6a which are "ON."

The second motor drive circuit 35 that constitutes a control section calculates a current value to transmit rotation (torsion) corresponding to the calculated torque to the insertion portion 10a and supplies the calculated current value to the second motor 27 to rotate the insertion portion 10a.

The second motor 27 is driven to rotate according to the current value (drive signal) from the second motor drive circuit 35. Thus, the second motor 27 constitutes an insertion portion driving member configured to generate a drive force to rotate the insertion portion 10a pivotably held to the operation section 11.

The rotation drive force of the second motor 27 is transmitted to the insertion portion 10a via the gear 28, the gear 26 and the bearing 25. Thus, when the power spiral tube 17 receives the torque, the rotation (torsion) corresponding to the rotational force is transmitted to the insertion portion 10a, and the operator can intuitively realize the torque received by the power spiral tube 17.

Next, operation of the insertion apparatus configured as described above will be described.

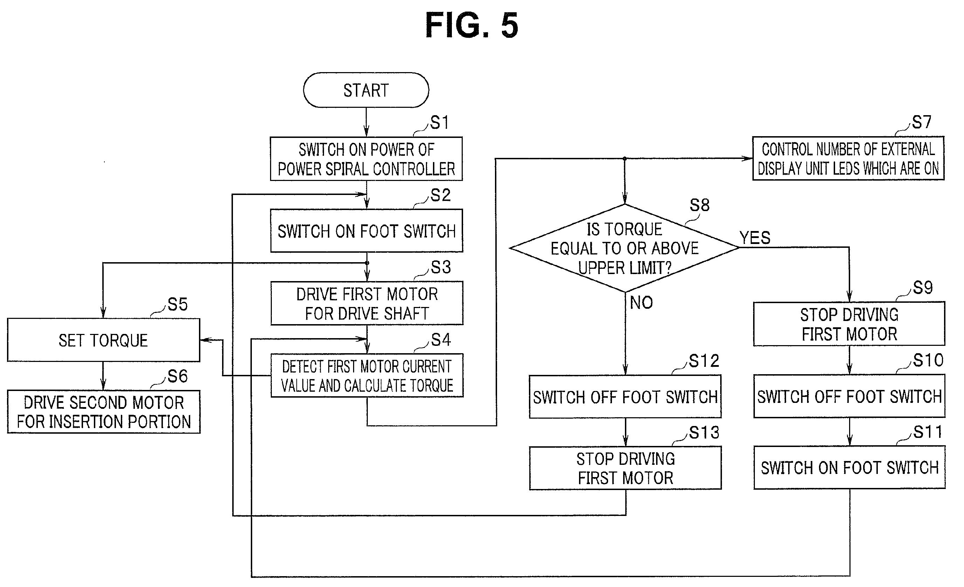

Fig. 5 is a flowchart for describing operation of the insertion apparatus.

First, power to the power spiral controller 3 is turned ON (step S1). Next, the foot switch 7 is turned ON (step S2). When the foot switch 7 is turned ON, an operation signal corresponding to the amount of depression is inputted to the first motor drive circuit 33 of the power spiral controller 3.

The first motor drive circuit 33 generates a current value (drive signal) corresponding to the operation signal, supplies the current value to the first motor 23 and drives the first motor 23 for the drive shaft 22 (step S3). Next, the torque detection circuit 34 detects the current value supplied to the first motor 23 and calculates the torque received by the power spiral tube 17 (step S4).

When the foot switch 7 is turned ON in step S2 and when the torque received by the power spiral tube 17 is calculated in step S4, the torque detection circuit 34 sets the detected calculated torque in the second motor drive circuit 35 (step S5). The second motor drive circuit 35 calculates a current value to transmit the rotation (torsion) corresponding to the calculated torque to the insertion portion 10a, supplies the current value to the second motor 27 for the insertion portion 10a and drives the second motor 27 for the insertion portion 10a (step S6). Thus, when the power spiral tube 17 receives the torque, the rotation (torsion) corresponding to the force is transmitted to the insertion portion 10a.

When the torque received by the power spiral tube 17 is calculated in step S4, the torque detection circuit 34 controls the number of LEDs 6a which are "ON" of the external display unit 6 in accordance with the calculated torque (step S7).

When the torque received by the power spiral tube 17 is calculated in step S4, the torque detection circuit 34 determines whether or not the calculated torque is equal to or above an upper limit value (step S8). When it is determined that the torque is equal to or above the upper limit value, the determination result is "YES" and the first motor drive circuit 33 stops driving the first motor 23 (step S9). When the driving of the first motor 23 is stopped, the operator turns OFF the foot switch 7 (step S10) and when the foot switch 7 is turned ON again (step S11), the flow returns to step S4 and repeats similar processes.

On the other hand, when it is determined that the torque is smaller than the upper limit value, the determination result is "NO" and when the operator turns OFF the foot switch 7 (step S12), the first motor drive circuit 33 stops driving the first motor 23 (step S 13). After that, the flow returns to step S2 and repeats similar processes.

As described above, the endoscope system 1 calculates torque received by the power spiral tube 17 using the torque detection circuit 34, causes the second motor drive circuit 35 to drive the second motor 27 with a current value corresponding to the torque so as to transmit the rotation corresponding to the torque received by the power spiral tube 17 to the insertion portion 10a.

While holding the operation section 11 of the endoscope 2 by the right hand and holding the insertion portion 10a by the left hand, for example, the operator inserts the insertion portion 10 into the body cavity. In this case, since the rotation corresponding to the torque received by the power spiral tube 17 is transmitted to the insertion portion 10a, the operator can realize the torque received by the power spiral tube by the left hand holding the insertion portion 10a.

Thus, the endoscope system which is the insertion apparatus of the present embodiment allows the operator to intuitively realize the torque received by the power spiral tube.

Note that the present embodiment is configured to rotate the insertion portion 10a in accordance with the torque received by the power spiral tube 17, but the present embodiment may also be configured, for example, to attach an overtube to the insertion portion 10a, not allow the insertion portion 10a to rotate and cause the overtube attached to rotate in accordance with the torque received by the power spiral tube 17.

Moreover, steps in the flowchart described in the Specification may be executed by changing the order of execution or a plurality of steps may be executed simultaneously or steps may be executed in different order every time the steps are executed, provided that it does not conflict with the nature thereof.

The present invention is not limited to the aforementioned embodiment, but can be modified or altered in various ways without departing from the spirit and scope of the present invention.

The present application claims priority based on Japanese Patent Application No. 2014-147066, filed on July 17, 2014, the disclosure of which is incorporated by reference in its entirety in the specification, claims and drawings of the present application.

## Claims

1. An insertion apparatus comprising:
an insertion portion configured to extend along a longitudinal axis from a proximal end direction to a distal end direction;
a rotation unit comprising a spiral fin portion configured to extend along the longitudinal axis in a spiral shape, the rotation unit being provided on an outer circumferential direction side of the insertion portion so as to be rotatable with respect to the insertion portion around the longitudinal axis;
a rotation unit driving member configured, by being driven, to generate a drive force to rotate the rotation unit;
an operation section provided on a proximal end side of the insertion portion;
an insertion portion driving member configured to generate a drive force to rotate the insertion portion pivotably held to the operation section;
a drive current calculation section configured to calculate a current value for driving the rotation unit driving member;
a torque calculation section configured to calculate torque to be added to the rotation unit from a calculation result of the drive current calculation section; and
a control section configured to perform control so as to supply a current to rotate the insertion portion driving member according to the calculation result of a torque calculation section.

2. The insertion apparatus according to claim 1, further comprising:
a bearing section provided in the operation section to which the insertion portion is pivotably held;
a gear member provided in the operation section and configured to transmit the drive force generated in the insertion portion driving member to the bearing section; and
a gear member for driving the insertion portion provided in the operation section, connected to the insertion portion driving member and configured to engage with the gear member.

3. The insertion apparatus according to claim 1, further comprising a rotation operation section configured to rotate the rotation unit,
wherein the drive current calculation section calculates a current value for driving the rotation unit driving member based on an amount of operation of the rotation operation section.

4. The insertion apparatus according to claim 1, wherein the torque calculation section determines whether or not the calculated torque is equal to or above an upper limit value, and stops, upon determining that the torque is equal to or above the upper limit value, generation of the drive force by the rotation unit driving member.

5. The insertion apparatus according to claim 4, wherein after the generation of the drive force is stopped, when the rotation operation section is operated from OFF to ON, the torque calculation section calculates torque to be added to the rotation unit.
